# EUROPEAN PATENT SPECIFICATION

(11) **EP 3 701 265 B1**
(45) Date of publication and mention of the grant of the patent: **11.09.2024**
(21) Application number: 18869602.5
(22) Date of filing: 25.10.2018
(51) Int. Cl.: G01N 33/574, C12N 5/22, G01N 21/00, G01N 33/567

(54) **METHOD FOR DIAGNOSING NEOPLASMS OF LYMPHOID TISSUE**
VERFAHREN ZUR DIAGNOSE VON NEOPLASMEN VON LYMPHOIDEM GEWEBE
PROCÉDÉ DE DIAGNOSTIC DE NÉOPLASMES DE TISSU LYMPHOÏDE

(30) Priority: 25.10.2017 PL 42326617
(43) Date of publication of application: 02.09.2020
(73) Proprietor: Uniwersytet Medyczny Im. Piastów Slaskich We Wroclawiu, 50-367 Wroclaw (PL)
(72) Inventor: DUS -SZACHNIEWICZ, Kamila, 54-104 Wroclaw (PL); WOZNIAK, Marta, 50-067 Wroclaw (PL); ZDUNIAK, Krzysztof, 50-349 Wroclaw (PL); WALASZEK, Kinga, 35-201 Rzeszów (PL); DROBCZYNSKI, Slawomir, 53-307 Wroclaw (PL)
(74) Representative: Witek, Rafal
(86) International application number: PCT/PL2018/000103
(87) International publication number: WO 2019/083383

(56) References cited:
- WO-A1-2016/154620
- CN-A- 102 747 156
- CN-A- 104 931 699
- BRONKHORST P. J. H. ET AL: "The mechanism of red cell (dis)aggregation investigated by means of direct cell manipulation using multiple optical trapping", BRITISH JOURNAL OF HAEMATOLOGY, vol. 96, no. 2, 1 February 1997 (1997-02-01), GB, pages 256 - 258, XP055805862, ISSN: 0007-1048, Retrieved from the Internet <URL:https://onlinelibrary.wiley.com/doi/pdfdirect/10.1046/j.1365-2141.1997.d01-2036.x> DOI: 10.1046/j.1365-2141.1997.d01-2036.x
- DUS-SZACHNIEWICZ KAMILA ET AL: "Differentiation of single lymphoma primary cells and normal B-cells based on their adhesion to mesenchymal stromal cells in optical tweezers", SCIENTIFIC REPORTS, vol. 9, no. 1, 1 December 2019 (2019-12-01), XP055805742, Retrieved from the Internet <URL:https://www.nature.com/articles/s41598-019-46086-y.pdf> DOI: 10.1038/s41598-019-46086-y
- GOU, X. ET AL.: "Cell adhesion manipulation through single cell assembly for characterization of initial cell -to- cell interaction", BIOMEDICAL ENGINEERING, vol. 14, 2015, pages 114, XP055597108
- HEINIG, K. ET AL.: "Access to Follicular Dendritic Cells Is a Pivotal Step in Murine Chronic Lymphocytic Leukemia B- cell Activation and Proliferation", CANCER DISCOVERY, vol. 4, no. 12, 24 September 2014 (2014-09-24), pages 1448 - 1465, XP055597128
- DROBCZYNSKI, S. ET AL.: "Real-time force measurement in double wavelength optical tweezers", JOURNAL OF THE OPTICAL SOCIETY OF AMERICA, vol. 34, no. 1, 1 January 2017 (2017-01-01), pages 38 - 43, XP055597117

## Description

The object of the invention is a method for identifying normal and neoplastic cells, in particular normal B lymphocytes and non-Hodgkin lymphoma cells in patients with suspected lymphoma. The invention belongs to the field of diagnostics of lymphoid tissue neoplasms.

Non-Hodgkin lymphomas (NHL) hold eighth position of all neoplasms among men and eleventh among women worldwide. It is estimated that lymphomas are diagnosed in more than 350,000 individuals annually. The number of deaths is about 200,000. In Poland, non-Hodgkin lymphomas constitute about 3% of cases and deaths due to neoplastic diseases among men and 2.5% among women according to the Polish National Cancer Registry, 2014. Many years of more and more detailed studies have revealed that under the same name, there are different subtypes of diseases present. Despite the fact that lymphomas initially might have similar clinical symptoms, genetic and molecular profiles thereof vary, and hence they differ in terms of prognosis, course and treatment.

Current diagnostic procedures used in patients with suspected lymphoproliferative disorder include pathomorphological analysis of tissue samples collected from the affected organ and flow cytometry of biopsy specimens.

In lymphoma diagnostics, histopathological analysis based on various materials depending on the clinical picture is performed as a standard. Sections collected surgically most frequently come from lymph nodes or extranodal tissues: skin, gastrointestinal tract, bone marrow, spleen, thymus and tonsils. In most cases, in order to specify the type of lymphoma, it is necessary to perform at least one additional testing: immunohistochemical, genetic or molecular.

Immunohistochemical staining is the differential diagnostics of lymphomas based on the National Comprehensive Cancer Network. The interpretation of immunophenotype should correlate with clinical picture and morphological features of lymphomas.

In flow cytometry, a material from fine-needle aspiration biopsy of a lymph node, peripheral blood or bone marrow is subjected to immunophenotyping.

Lymphoma diagnostics also includes cytogenetic testing (*in situ* hybridisation/FISH) and cytology testing, in which a material from fine-needle aspiration biopsy of a lymph node, peripheral blood or bone marrow is analysed, as well as diagnostic imaging which employs tomographic techniques.

In the state of art, some other attempts of providing an effective diagnostic test which could be used in case of lymphoproliferative disorders are known.

EP2697256 provides a method for diagnosing lymphomas based on the detection of LY6G6F, VSIG10, TMEM25 and LSR proteins expression.

US4038145 discloses a diagnostic method in which the mixed lymphocyte reaction (MLR) is used in tissue culture to detect the tumour-induced autoimmune reaction characteristic of lymphoid neoplasms. Lymphocytes from blood, spleen and lymph nodes are used in combination. The mixed lymphocyte reaction resulting from any of three possible combinations indicates the presence of a lymphoid neoplasm.

EP3221467 discloses a diagnostic method for detecting lymphomas, comprising the following steps: determining the hGSTA1 expression level in a tumor sample obtained from a patient, ii) comparing the expression level determined in step i) with a predetermined reference value, iii) concluding that the patient is affected when the hGSTA1 expression level is lower than the predetermined reference value.

EP3167288 relates to methods for diagnosing hematological cancers. Particularly, the invention relates to a method comprising the steps of i) detecting the presence of CD45RARO NK cells in a sample obtained from a patient, ii) concluding that the patient suffers from a haematological cancer when the presence of CD45RARO NK cells is detected in the sample, wherein the presence of at least one phenotypic marker indicates the type of the cancer.

EP2612869 provides an antibody with the ability to bind the ED-A isoform of fibronectin, wherein the antibody binding with the protein indicates the presence of neoplastic cells of lymphoid tissue.

US2011287948 discloses a method for diagnosing neoplasms, including lymphomas, based on algorithm taking into account a measurement of adhesion forces between abnormal cells using atomic force microscope (AFM) or optical tweezers.

EP2624742 provides a diagnostic method in which mechanical properties of tumour cells and reference cells are analyzed under a mechanical load which leads to linear or non-linear deformation of said cell. Cells expansion, which results from the application of mechanical stress, is used to determine the risk of tumour metastases, as well as, where appropriate, the presence of uncontrollably proliferating invasive cells or tissue of tumour origin. In case of non-linear deformation of the cell, the risk of the presence of abnormally proliferating cells is determined, based on the mean value of the expansion in the direction of stressing of cells in the sample. The method is realised using optical tweezers.

Gou X. et al., 2015 "Cell adhesion manipulation through single cell assembly for characterization of initial cell-to-cell interaction" reports a case study of characterizing initial interaction between leukemia cancer cells and bone marrow stromal cells through the use of an optical tweezers-based cell manipulation tool. The document does not disclose a diagnostic assays.

Heinig K. et al., 2014 "Access to follicular dendritic cells is a pivotal step in murine chronic lymphocytic leukemia B-cell activation and proliferation" reports that CLL and other indolent lymphoma are not curable and usually relapse after treatment, a process in which the tumor microenvironment plays a pivotal role. The authors dissect the consecutive steps of CXCR5-dpendent tumor cell lodging and LTβR-dependent stroma-leukemia cell interaction. The publication reports that leukemia B cells need a longer time to form stable adhesion with stromal cells but there the investigation period is 30 minutes. No diagnostic assay is disclosed.

Drobczynski S. et al., 2017 "Real-time force measurement in double wavelength optical tweezers" shows a procedure for mapping the trap stiffness in optical tweezers over the whole sample area. Such a map allows development of a real -time procedure for force mesarument at any point of the sample area suitable for measuring forces in living cells samples. The document does not disclose a diagnostic assays.

In Bronkhorst P.J.H. et al., 1997 "The mechanism of red cell (dis)aggregation investigated by means of direct cell manipulation using multiple optical trapping" multiple optical trapping to study the mechanism of red cell (dis)aggregation is used. Two sets of optical 'tweezers' were used to bring two red blood cells together to form a two-cell aggregate and then to pull them apart, to study the interaction between the cells. It is reported that cross-bridging occurred in normal reversible aggregation as binding and the occurrence of small tethers between opposite cell membranes was observed. The publication discloses that the formation of adhesion is in the order of seconds rather than minutes. The document does not disclose a diagnostic assays.

The percentage of patients with reactive changes among all patients diagnosed with a proliferative disorder is about 30%. The diagnostic process requires more and more complicated diagnostic methods to accurately classify as well as quickly and effectively differentiate between neoplastic changes and non-malignant reactive changes.

Despite available diagnostic methods for lymphoid tissue neoplasms in the state of art, there is still a need to provide a simple and highly specific method for identifying abnormal cells and normal cells in patients with suspected neoplasm.

The aim of the invention is to provide an effective method enabling the efficient identification of abnormal cells formed in the process of carcinogenesis in patients with a suspected neoplastic disease of lymphoid tissue.

The aim has been achieved by the present invention.

The object of the invention is a method for diagnosing neoplasms of lymphoid tissues including the steps of:
a) providing B lymphocytes previously collected and isolated from a patient,
b) preparing a suspension of B lymphocytes as well as bone marrow stromal cells for a measuring procedure,
c) by cell manipulation technique, bringing a B lymphocyte cell into contact with a bone marrow stromal cell such that the B lymphocyte and the bone marrow stromal cell remain in a direct contact until a stable bond is formed,
d) measuring time of the formation of the stable adhesion (t) between the B lymphocyte cell and the bone marrow stroma cell, wherein the stable adhesion formation of t > 60 s indicates the presence of neoplastic cells.

The cell manipulation technique in steps b) and c) is optical tweezers. Preferably, before bringing the B lymphocyte into contact with the bone marrow stromal cell, the B lymphocyte is captured into an optical trap by optical tweezers.

Preferably, the B lymphocyte is captured into the optical trap with the force of 100 pN. Preferably, the bond is considered as stable, when the B lymphocyte cannot be pulled away from the bone marrow stromal cell using the optical trap generated with a laser power of 200 mW.

Preferably, the neoplasm of lymphoid tissue is B-cell non-Hodgkin lymphoma (NHL). Preferably, the neoplasm of lymphoid tissue is diffuse large B-cell lymphoma (DLBCL), follicular lymphoma (FL), mantle cell lymphoma (MCL), MALT lymphoma, Burkitt lymphoma (BL), and high grade B-cell lymphoma (HGBL).

Preferably, the B lymphocyte cell is brought into contact with the central part of the bone marrow stromal cell.

Preferably, the bone marrow stromal cell is a fibroblast.

Preferably, bone marrow stroma cells are of HS-5 line.

Optical tweezers are a device that generates optical forces able to manipulate with objects sized between 0.4 µm to 20.0 µm on a microscope slide. The appropriate shape of the laser beam with the wavelength of infrared or visible light range enables the analysis of mechanical properties of cells, the determination of elasticity of cell membranes, intercellular interactions in co-cultures of healthy and neoplastic cells, biological materials, DNA strands. The optical trap generated by the laser beam shaping system, coming from a highly focused laser beam, captures objects present on the slide in the focal plane of the microscopic lens. The system of optical tweezers allows direct or indirect (through special dielectric nanoparticles) measurements of shifts, forces affecting biological structures, or adhesive properties of captured cells.

In the method according to the invention, other cell manipulation techniques can be used.

The essence of the invention relates to the identification of normal and abnormal B lymphocytes based on a time necessary for a lymphoma cell to form a stable adhesion with bone marrow stromal fibroblasts. The method according to the invention enables the effective diagnostics of the neoplasm by differentiating between normal cells and abnormal cells of a stable co-culture of fibroblasts and B lymphocytes in optical tweezers. Thus, the method according to the invention based on the interactions between B lymphocytes and bone marrow stroma fibroblasts enables the distinction between normal B lymphocytes and abnormal cells with high effectiveness. Therefore, the diagnostic procedure at the first step thereof is already shorter and cheaper.

### Description of Figures

Fig. 1 shows the steps of sliding the B lymphocyte cell over the fibroblast cell of HS-5 line. a) moving the cell above the substrate; no direct contact between the cells, b) formation of a contact site between B lymphocyte cell membrane and the surface of HS-5 cell; the lymphocytic cell remains exactly in the centre of the optical trap, c) moving the B lymphocytes to the central part of the HS-5 cell.
Fig. 2 shows a) abnormal B lymphocyte captured in the optical trap, b) lymphoma cell brought into contact with HS-5 bone marrow stroma cell, with the contact maintained in the optical trap.
Fig. 3 shows the number of patients varying in terms of sex and health condition, as well as the results from the test of independence.
Fig. 4 shows the time of formation of a strong bond between cells in test and control groups, as well as the results from the Mann-Whitney U test.
Fig. 5 shows a ROC curve of the time of formation of a strong bond between cells, as well as the area under the ROC curve (AUC = 0.997).
Fig. 6 shows a histogram of the time of formation of a strong bond between abnormal and normal cells, as well as test sensitivity (Sens.) and specificity (Spec.) for a cut-off value (cut-off) of t > 60 s.
Fig. 7 shows the minimal number of isolated cells required as a function of test power.

### Example

### Clinical material

The aim of the inventors was to obtain cells in a state as close as possible to the state thereof in *in vivo* conditions. The method according to the invention utilised a specifically defined clinical material in a form of normal and neoplastic B lymphocytes obtained for routine cancer diagnostics purposes. The clinical material comprises neoplastic cells obtained from patients diagnosed with diffuse large B-cell lymphoma (DLBCL), follicular lymphoma (FL), mantle cell lymphoma (MCL), mucosa associated lymphoid tissue lymphoma (MALT) , Burkitt lymphoma (BL), and high grade B-cell lymphoma (HGBL). Information on the origin and type of the clinical material included in the study are collected in Tables 1 and 2, wherein Table 2 shows clinical and pathological data for control patients included in the study. The study included 47 individuals aged between 21 and 89 years old (mean M = 59.8; standard deviation SD = 15.8 years). Basic statistics of traits characteristic for both groups are presented in Table 3 (age and sex of patients in test and control group; results of Mann-Whitney U test). The statistical analysis of results was performed in Statistica 11 software, StatSoft Poland. Both groups of patients did not differ significantly in terms of age (59.2 vs. 61.1 years; p = 0.699), nor sex structure (percentage of women: 50.0% vs. 66.7%; p = 0.449).

**Table 1**

| **NO.** | **SEX** | **AGE** | **DIAGNOSIS** | **MATERIAL** | **SITE** | **IMMUNOPHENOTYPE** |
|---|---|---|---|---|---|---|
| 1 | M | 50 | **Extranodal DLBCL, NOS, non GCB** | B | right cervical lymph node, pubic symphysis infiltration | FC: 42% of abnormal B lymphocytes. Double expressor lymphoma: BC12+/-/BC16+/MYC-. |
| 2 | M | 75 | **Extranodal DLBCL, NOS, non GCB** | B | pubic symphysis infiltration | FC: 56% of abnormal B lymphocytes, Double expressor lymphoma: BCI2+ higher/BC16-/+/MYC-. |
| **3** | F | 46 | **Extranodal DLBCL, NOS, GCB.** | B | left supraclavicular lymph node | FC: 96% of abnormal B lymphocytes, Triple expressor lymphoma: BCI2+ higher/BC16+/MYC+. |
| **4** | F | 61 | **Extranodal DLBCL, NOS, GCB** | B | left thigh tumour below inguinal ligament | FC: 91% of abnormal B lymphocytes, Triple expressor lymphoma: BCL2+higher/ BCL6+high/MYC+. |
| **5.** | F | 54 | **Nodal DLBCL, NOS, GCB** | B | right submandibular lymph node | FC: 41% of abnormal B lymphocytes, Double expressor lymphoma: BCL2-/BCL6+high/MYC+. |
| **6.** | M | 62 | **Nodal DLBCL, NOS, GBC** | B | right cervical lymph node | FC: 63% of abnormal B lymphocytes, Double expressor lymphoma: BCL2+higher/ BCL6+/MYC-/+. |
| **7.** | F | 55 | **Extranodal DLBCL, NOS, GCB** | B | skin-infiltrating tumour of abdominal cavity | FC: 24% of abnormal B lymphocytes, Double expressor lymphoma: BCL2+/BCL6+/MYC-. |
| **8.** | M | 44 | **Nodal DLBCL, NOS, GCB** | B | left cervical lymph node | FC: 34% of abnormal B lymphocytes, Double expressor lymphoma BCL2+/BCL6+/MYC-. |
| **9.** | F | 54 | **Nodal DLBCL, NOS, non-GCB** | B | left cervical lymph node, nodal masses in abdominal cavity | FC: 50% of abnormal B lymphocytes, Triple expressor lymphoma BC12+/BC16+/MYC+. |
| **10.** | M | 32 | **Nodal DLBCL, NOS, between GCB and non-GCB** | B | cervical lymph node | FC: 23% of abnormal B lymphocytes, One expressor lymphoma: BCL2-/ BCL6+/MYC-. |
| **11.** | F | 61 | **Nodal DLBCL, NOS,** | B | right lymph node near sternocleidomastoid muscle | FC: 24% of abnormal B lymphocytes. Double expressor lymphoma: BCL2-/ BCL6+/MYC-/+. |
| | | | **GCB** | | | |
| **12.** | F | 74 | **Primary cutaneous DLBC, GCB with BL** | B | skin lesions on right lower leg, lower part of thigh with ulcers | FC: 45% of abnormal B lymphocytes, Double expressor lymphoma: BC12-/BC16+/MYC+/. |
| **14.** | F | 84 | **HGBL, NOS** | B | right side nasal cavity tumour | FC: 44% of abnormal B lymphocytes. Triple expressor lymphoma: BCL2+higher/BCL6+/MYC+. |
| | | | **between GCB and non-GCB** | | | |
| **15.** | M | 37 | **HGBL, GCB** | B | right bulky cervical lymph node | FC: 66% of abnormal B lymphocytes, Double expressor lymphoma: BCL2+higher/ BCL6+/MYC-. |
| **16.** | M | 60 | **HGBL, NOS** | B | right side calf skin tumours | FC: 97% of abnormal B lymphocytes, Triple expressor lymphoma BC12+/BC16+/MYC+. |
| **17.** | F | 70 | **FL** | B | abdominal cavity tumour | FC: 86% of abnormal B lymphocytes, WHO classification G2 low grade. |
| **18.** | F | 79 | **FL** | B | abdominal lymph nodes | FC: 65% of abnormal B lymphocytes, |
| **19.** | F | 49 | **FL** | B | supraclavicular lymph node | FC: 24% of abnormal B lymphocytes, WHO classification G2 low grade. |
| **20.** | F | 66 | **FL** | S | iliac lymph nodes | FC: 86% of abnormal B lymphocytes, WHO classification G1 low grade. |
| **21.** | M | 65 | **FL** | S | cervical lymph node | FC: 53% of abnormal B lymphocytes, WHO classification G2 low grade. |
| **22.** | M | 56 | **FL** | B | left inguinal lymph node | FC: 42% of abnormal B lymphocytes, WHO classification G2 low grade. |
| **23.** | M | 32 | **FL** | B | bulky abdominal cavity tumour | FC: 95% of abnormal B lymphocytes, Acc WHO classification G1. |
| **24.** | M | 70 | **FL** | B | left cervical lymph node | FC: 19% of abnormal B lymphocytes, WHO- no data |
| **25.** | F | 67 | **FL** | S | lymph node of supraclavicular tissue | FC: 47% of abnormal B lymphocytes |
| **26.** | F | 86 | **MCL** | B | nodal lesion in abdominal cavity | FC: 96% of abnormal B lymphocytes |
| **27.** | F | 69 | **MCL** | B | right cervical lymph nodes | FC: 86.2% of abnormal B lymphocytes |
| **28.** | M | NA | **MCL** | S | gastric and duodenal mucosa | FC: 95% of abnormal B lymphocytes |
| **29.** | M | 64 | **MCL** | S | lymph node of inguinal area | FC: 94% of abnormal B lymphocytes |
| **30.** | M | 21 | **BL** | B | nodal tumour of right axilla | FC: 90% of abnormal B lymphocytes, BCL2-/BCL6-/ MYC+. |
| **31.** | M | 54 | **BL** | B | right cervical node | FC: 65% of abnormal B lymphocytes |
| **32.** | M | 65 | **MALT** | B | Right breast tumour | FC: 80% of abnormal B lymphocytes |
| | | | | | | |

| | | | | | | |
|---|---|---|---|---|---|---|
| F-female, M-male, B-biopsy material, S-surgical material | | | | | | |

**Table 2**

| **NO.** | **SEX** | **AGE** | **DIAGNOSIS** | **SITE** |
|---|---|---|---|---|
| **1.** | F | 82 | **Cholecystitis acuta** | common bile duct area |
| **2.** | F | 76 | **Cholecystitis acuta** | common bile duct area |
| **3.** | F | 65 | **Cholecystitis acuta** | common bile duct area |
| **4.** | F | 80 | **Cholecystitis acuta** | common bile duct area |
| **5.** | M | 65 | **Colitis chronica** | small intestine mesenteric lymph node |
| **6.** | F | 45 | **Pancreatitis acuta** | small intestine mesenteric lymph node |
| **7.** | M | 29 | **Lymphonodulitis reactiva** | cervical lymph node |
| **8.** | F | 51 | **Lymphonodulitis reactiva** | lymph node of left femoral triangle area |
| **9.** | F | 58 | **Lymphonodulitis reactiva** | cervical lymph node |
| **10.** | M | 43 | **Lymphonodulitis reactiva** | cervical lymph node |
| **11.** | M | 74 | **Lymphonodulitis reactiva** | right inguinal node |
| **12.** | F | 44 | **Lymphonodulitis reactiva** | lymph node of submandibular tissue |
| **13.** | F | 72 | **Lymphonodulitis reactiva** | axillary node |
| **14.** | M | 89 | **Adenocarcinoma coli, GII** | large intestine mesentery |
| **15.** | F | 44 | **Invasive carcinoma of no special type (NST)** | axillary node |

| | | | | |
|---|---|---|---|---|
| F - female, M - male | | | | |

**Table 3**

| Trait (variable) | | All patients | | Group of patients | | | | T vs. C |
|---|---|---|---|---|---|---|---|---|
| | | | | Test (T) | | Control (C) | | |
| | | *N* = 47 | | *N* = 32 | | *N* = 15 | | P |
| Age (in years) | | | | | | | | 0.699 |
| | *M* ± *SD* | 59.8 ± 15.8 | | 59.2 ± 15.1 | | 61.1 ± 17.8 | | |
| | *Me* [Q₁; *Q*₃] | 61 [49; 72] | | 61 [52; 70] | | 65 [44; 76] | | |
| | *Min* - *Max* | 21-89 | | 21 - 86 | | 29-89 | | |
| Sex: | | *n* | % | *n* | % | *n* | % | 0.449 |
| | Women | | | 16 | 50.0 | 10 | 66.7% | |
| | Men | | | 16 | 50.0 | 5 | 33.3% | |

### Protocol for clinical material collection

### Biopsies

Cell suspension is collected under USG control to PBS solution pH=7.4 (Life Technologies, cat. No. 10010-023), and subsequently transferred sterilely to a probe with EDTA (Becton Dickinson, EDTA 7.2 mg, cat. No. 368861). After 40 minutes, the material is deep-frozen in 30% foetal bovine serum (Life Technologies, FBS, cat. No. 10270-098) and 10% DMSO (BioShop, cat. No. DMS555). Cells are kept in liquid nitrogen.

### Surgical material

Following the surgical lymph node collection, the material is transferred to sterile PBS solution with 2% antibiotic and antimycotic (Life Technologies, cat. No. 15240062). B lymphocytes are isolated from the lymph node up to 4 hours post collection. The cells are aspirated using a biopsy needle to a syringe with the anticoagulant and sterile PBS solution. After 40 minutes of incubation at the room temperature, the material is frozen in 30% FBS and 10% DMSO. Cells are kept in liquid nitrogen.

### Preparation of clinical samples for measurements in optical tweezers

The holographic system of tweezers used in the present method has the following parameters: trapping lasers: 1064 nm 4W, 980 nm 900 mW; microscopic lens 100x NA 1.3; holographic generation of optical traps using Hamamatsu LCoS modulator with the resolution of 800x600; laser beam control using galvano mirrors; optical path for spectroscopic analysis; video camera: CMOS max frame rate 10000 fps (frames per second).

The sample is thawed, centrifuged twice in RPMI medium (1800 × g for 7 min). The number and viability of cells is determined, and then the cells are incubated for 24 h at 37°C, 5% CO₂.

In cases where the percentage of abnormal B lymphocytes in immunophenotyping (flow cytometry) was lower than 90%, the depletion of T lymphocytes and NK cells was performed using magnetic beads coated with anty-CD3 antibody (CD3+ Manual MACS^{®} Cell Separation, Miltenyi Biotech). Control samples are subjected to this procedure each time. Samples of patients No. 7, 11, 24 due to a large percentage of normal B lymphocytes were subjected to additional magnetic isolation using beads coated with CD5+ and CD10+ antibodies. The effectiveness of magnetic isolation was confirmed with flow cytometry. Just before measurements in optical tweezers, cells are centrifuged and a suspension with density of 5×10⁵/ml is prepared.

### Bone marrow stromal cell culture

In order to obtain optimal and replicable experimental conditions, a protocol has been developed which determines the method for starting a culture of HS-5 bone marrow stromal cells. For HS-5 cell line culture (ATCC, cat. No. ATCC^{®} CRL-11882^{™}), DMEM medium is recommended (ATCC, Dulbecco's modified Eagle's medium, cat. No. ATCC^{®} 30-2002^{™}). The medium was additionally supplemented with foetal bovine serum (ATCC, FBS, cat. No. ATCC^{®} 30-2020^{™}). Cell culture was maintained in 37°C at humid atmosphere in the presence of 5% CO₂.

HS-5 cells after reaching the confluence of 80% in a culture flask were passaged and their quantity and viability were determined using Trypan Blue (Invitrogen, The Countess^{™} automated cell counter). Subsequently, the cells in experimentally defined quantity (25,000 cells in 200 ul of DMEM medium) were seeded on a sterile Petri dish with a glass bottom (Greiner bio-one, cat. No. 627960). After 24 h from starting the culture, 1 ml of culture medium was added to cells. After 72 h from starting the culture, the confluence of morphologically mature cells on dishes was about 60%. Just before measurements in optical tweezers, cells not adhering to the substrate were washed off.

### Measuring procedure

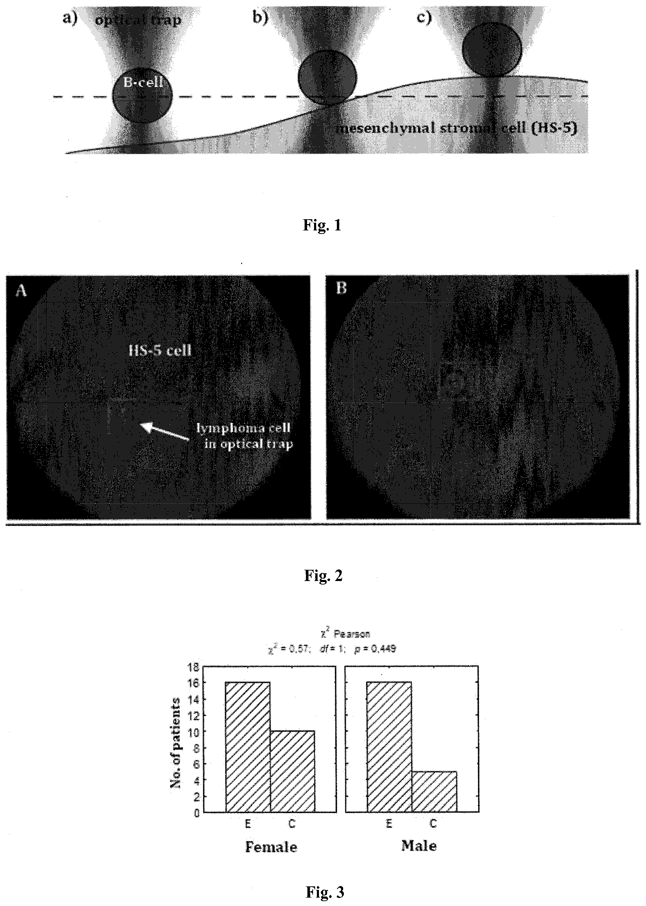

A measurment dish with HS-5 fibroblasts was placed on a table of the optical manipulator. 100 ul of B lymphocyte suspension was transferred directly into the dish. Time of cell sedimentation was about 5 minutes. Subsequently, the B lymphocyte was captured into the optical trap with the force of 100 pN and brought into contact with the central part of the stromal cell, such that the cells remained in a direct contact until the stable co-culture formation (Fig. 1).

Experimentally determined time intervals for cell trapping were, respectively: 5, 10, 15, 20, 40, 60 and 90 s for normal cells and 30, 40, 60, 90, 120, 150, 180, 210, 240, 270, 300, 360, 420 and 480 s for abnormal cells.

It has been assumed that a stable co-culture is a state in which the B lymphocyte cannot be pulled away from HS-5 stromal cell using the optical trap generated with a laser power of 200 mW.

The dish with cell substrate was changed every 20 minutes of manipulation while not allowing for the temperature of the culture medium to drop below 33°C. The experiment was repeated at least twice for each case.

Tables 4 and 5 show the measurement results of time required for the co-culture formation in optical tweezers for abnormal and normal cells, respectively. Table 4 shows the time required for the formation of a stable bond between abnormal B lymphocytes and HS-5 stromal cells using optical tweezers. Table 5 shows the time required for the formation of a stable bond between normal B lymphocytes and HS-5 stroma cells using optical tweezers.

**Table 4**

| **NO.** | **NO. OF MEASUREMENTS** | **MEAN [s]** | **SD** | **RANGE [s]** | **NO.** | **NO. OF MEASUREMENTS** | **MEAN [s]** | **SD** | **RANGE [s]** |
|---|---|---|---|---|---|---|---|---|---|
| **1** | 73 | **131.5** | 49.4 | 60-210 | **17** | 62 | **202.2** | 64.0 | 120-360 |
| **2** | 73 | **162.2** | 59.2 | 90-270 | **18** | 54 | **120.5** | 46.0 | 60-240 |
| **3** | 80 | **157.9** | 57.3 | 90-300 | **19** | 56 | **304.8** | 59.4 | 210-420 |
| **4** | 76 | **168.9** | 55.8 | 90-270 | **20** | 65 | **180.0** | 50.3 | 90-270 |
| **5** | 54 | **160.5** | 48.4 | 90-300 | **21** | 72 | **109.2** | 32.4 | 60-180 |
| **6** | 60 | **339.5** | 65.4 | 210-480 | **22** | 55 | **317.4** | 86.1 | 120-420 |
| **7** | 59 | **163.2** | 44.7 | 90-300 | **23** | 53 | **110.9** | 28.9 | 60-150 |
| **8** | 53 | **162.4** | 43.0 | 120-360 | **24** | 57 | **201.6** | 69.4 | 120-360 |
| **9** | 52 | **264.2** | 52.1 | 120-360 | **25** | 68 | **157.1** | 60.9 | 60-300 |
| **10** | 65 | **167.1** | 49.7 | 90-300 | **26** | 62 | **119.0** | 44.8 | 60-210 |
| **11** | 52 | **209.4** | 54.3 | 120-300 | **27** | 51 | **120.0** | 32.3 | 60-180 |
| **12** | 64 | **257.8** | 68.2 | 120-360 | **28** | 50 | **115.2** | 41.7 | 60-210 |
| **13** | 47 | **324.2** | 59.2 | 210-420 | **29** | 58 | **124.1** | 33.6 | 60-180 |
| **14** | 58 | **300.5** | 61.4 | 180-420 | **30** | 72 | **268.7** | 62.9 | 180-420 |
| **15** | 54 | **336.7** | 85.2 | 180-480 | **31** | 58 | **315.0** | 73.9 | 180-420 |
| **16** | 67 | **265.1** | 94.8 | 120-420 | **32** | 42 | **350.7** | 53.8 | 270-420 |

**Table 5**

| **NO.** | **NO. OF MEASUREMENTS** | **MEAN [s]** | **SD** | **RANGE [s]** | **NO.** | **NO. OF MEASUREMENTS** | **MEAN [s]** | **SD** | **RANGE** |
|---|---|---|---|---|---|---|---|---|---|
| **1** | 93 | **17.1** | 8.9 | 5-30 | **9** | 98 | **16.7** | 8.3 | 5-40 |
| **2** | 74 | **18.5** | 8.1 | 5-40 | **10** | 54 | **37.1** | 21.4 | 10-90 |
| **3** | 67 | **29.6** | 12.1 | 10-60 | **11** | 78 | **36.2** | 20 | 15-90 |
| **4** | 48 | **41.2** | 24.4 | 10-90 | **12** | 68 | **23.8** | 11.7 | 5-60 |
| **5** | 55 | **33.4** | 13.9 | 10-60 | **13** | 51 | **42.3** | 11.1 | 30-60 |
| **6** | 57 | **49.8** | 17 | 20-90 | **14** | 70 | **21.8** | 7.2 | 10-40 |
| **7** | 98 | **18.4** | 9.9 | 5-60 | **15** | 66 | **20.4** | 10.3 | 5-40 |
| **8** | 110 | **22.4** | 13.1 | 5-60 | | | | | |

Basic statistics for the time of the stable adhesion formation for cells isolated from affected (T) and healthy individuals (C) together with the significance test are presented in Table 6.

**Table 6**

| Time of stable bond formation (s) | All cells | Cells | | Tvs. C |
|---|---|---|---|---|
| | | abnormal (T) | normal (C) | |
| | *N* = 2999 | *N* = 1912 | *N* = 1087 | *p* |
| *M* ± *SD* | 140.8 ± 116.0 | 205.6 ± 96.7 | 26.7±16.6 | |
| *Me* [*Q*₁; *Q*₃] | 120 [30; 210] | 180 [120; 270] | 20 [15; 30] | **<0.0001** |
| *Min* - *Max* | 5 - 480 | 60 - 480 | 5 - 90 | |

| | | | | |
|---|---|---|---|---|
| *M* - mean, *SD* - standard deviation, *Me* - median, *Q*₁ - lower quartile (25th percentile), *Q*₃ - upper quartile (75th percentile), *Min* - minimum, *Max* - maximum | | | | |

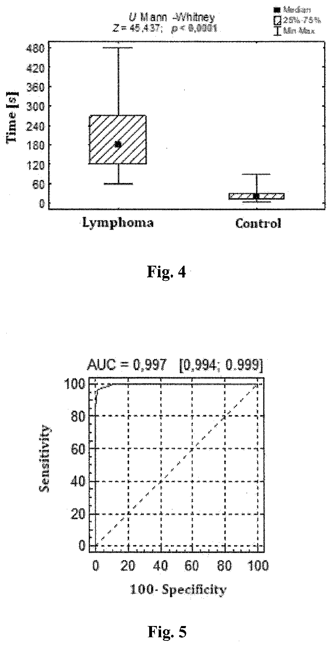

The obtained measurements indicate that lymphoma cells form a stable bond with HS-5 stroma fibroblasts from 2.2 to 20.5 times slower than normal cells. The time required for the stable bond formation in abnormal cells is significantly longer than in normal cells (180 s vs. 20 s). The result of the comparison between mean time values is statistically significant at the level of p < 0.001. The power of the test is close to one (Fig. 4).

Tables 7 and 8 show the percentage of B lymphocytes forming stable co-cultures with stromal cells in particular time intervals. Table 7 comprises the % distribution of abnormal cells forming a stable bond with stroma cells in particular time intervals. Table 8 comprises the % distribution of normal cells forming a stable bond with stroma cells in particular time intervals.

**Table 7**

| **PATIENTS** | | | | | | | | | | | | | | | | |
|---|---|---|---|---|---|---|---|---|---|---|---|---|---|---|---|---|
| **[s]** | **1** | **2** | **3** | **4** | **5** | **6** | **7** | **8** | **9** | **10** | **11** | **12** | **13** | **14** | **15** | **16** |
| **[%]** | | | | | | | | | | | | | | | | |
| **30** | 0 | 0 | 0 | 0 | 0 | 0 | 0 | 0 | 0 | 0 | 0 | 0 | 0 | 0 | 0 | 0 |
| **60** | 12.3 | 0 | 0 | 0 | 0 | 0 | 0 | 0 | 0 | 0 | 0 | 0 | 0 | 0 | 0 | 0 |
| **90** | 24.7 | 20.3 | 18.8 | 11.8 | 11.1 | 0 | 3.4 | 11.3 | 0 | 4.6 | 0 | 0 | 0 | 0 | 0 | 0 |
| **120** | 20.5 | 25.0 | 27.5 | 26.3 | 24.1 | 0 | 30.5 | 22.6 | 1.9 | 32.3 | 13.5 | 6.3 | 0 | 0 | 0 | 4.5 |
| **150** | 12.3 | 6.3 | 13.8 | 13.2 | 24.1 | 0 | 23.7 | 5.7 | 0 | 13.8 | 9.6 | 0 | 0 | 0 | 0 | 11.9 |
| **180** | 15.1 | 21.9 | 15.0 | 13.2 | 13.0 | 0 | 15.3 | 41.5 | 5.8 | 18.5 | 11.5 | 15.6 | 0.0 | 5.2 | 1.9 | 16.4 |
| **210** | 15.1 | 0 | 7.5 | 13.2 | 18.5 | 5.1 | 18.6 | 11.3 | 19.2 | 18.5 | 26.9 | 9.4 | 6.4 | 1.7 | 5.6 | 9.0 |
| **240** | 0 | 21.9 | 11.3 | 15.8 | 7.4 | 0 | 6.8 | 7.5 | 15.4 | 9.2 | 17.3 | 15.6 | 0 | 17.2 | 11.1 | 14.9 |
| **270** | 0 | 4.7 | 3.8 | 6.6 | 0 | 15.3 | 0 | 0 | 13.5 | 0 | 11.5 | 15.6 | 17.0 | 24.1 | 16.7 | 0 |
| **300** | 0 | 0 | 2.5 | 0 | 1.9 | 30.5 | 1.7 | 0 | 32.7 | 3.1 | 9.6 | 20.3 | 34.0 | 20.7 | 18.5 | 6.0 |
| **330** | 0 | 0 | 0 | 0 | 0 | 1.7 | 0 | 0 | 5.8 | 0 | 0 | 0 | 6.4 | 0 | 0 | 0 |
| **360** | 0 | 0 | 0 | 0 | 0 | 18.6 | 0 | 0 | 5.8 | 0 | 0 | 17.2 | 17.0 | 20.7 | 9.3 | 28.4 |
| **390** | 0 | 0 | 0 | 0 | 0 | 0 | 0 | 0 | 0 | 0 | 0 | 0 | 0 | 0 | 0 | 0 |
| **420** | 0 | 0 | 0 | 0 | 0 | 28.8 | 0 | 0 | 0 | 0 | 0 | 0 | 19.1 | 10.3 | 27.8 | 9.0 |
| **480** | 0 | 0 | 0 | 0 | 0 | 0 | 0 | 0 | 0 | 0 | 0 | 0 | 0 | 0 | 9.3 | 0 |

| **PATIENTS** | | | | | | | | | | | | | | | | |
|---|---|---|---|---|---|---|---|---|---|---|---|---|---|---|---|---|
| **[s]** | **17** | **18** | **19** | **20** | **21** | **22** | **23** | **24** | **25** | **26** | **27** | **28** | **29** | **30** | **31** | **32** |
| **[%]** | | | | | | | | | | | | | | | | |
| **30** | 0 | 0 | 0 | 0 | 0 | 0 | 0 | 0 | 0 | 0 | 0 | 0 | 0 | 0 | 0 | 0 |
| **60** | 0 | 20.4 | 0 | 0 | 16.7 | 0 | 13.2 | 0 | 7.4 | 18.0 | 5.9 | 14.0 | 5.2 | 0 | 0 | 0 |
| **90** | 0 | 11.1 | 0 | 3.1 | 27.8 | 0 | 26.4 | 0 | 11.8 | 27.9 | 29.4 | 34.0 | 25.9 | 0 | 0 | 0 |
| **120** | 19.4 | 46.3 | 0 | 24.6 | 34.7 | 5.5 | 37.7 | 22.8 | 23.5 | 21.3 | 31.4 | 26.0 | 34.5 | 0 | 0 | 0 |
| **150** | 11.3 | 1.9 | 0 | 12.3 | 16.7 | 0 | 22.6 | 8.8 | 8.8 | 8.2 | 23.5 | 16.0 | 19.0 | 0 | 0 | 0 |
| **180** | 19.4 | 9.3 | 0 | 16.9 | 4.2 | 7.3 | 0 | 22.8 | 30.9 | 21.3 | 9.8 | 0 | 15.5 | 8.3 | 0 | 0 |
| **210** | 11.3 | 11.1 | 1.8 | 21.5 | 0 | 5.5 | 0 | 8.8 | 5.9 | 3.3 | 0 | 10.0 | 0 | 13.9 | 10.3 | 0 |
| **240** | 24.2 | 0 | 23.2 | 15.4 | 0 | 5.5 | 0 | 22.8 | 2.9 | 0 | 0 | 0 | 0 | 29.2 | 6.9 | 0 |
| **270** | 3.2 | 0 | 21.4 | 6.2 | 0 | 3.6 | 0 | 1.8 | 2.9 | 0 | 0 | 0 | 0 | 16.7 | 5.2 | 7.1 |
| **300** | 6.5 | 0 | 19.6 | 0 | 0 | 16.4 | 0 | 3.5 | 5.9 | 0 | 0 | 0 | 0 | 13.9 | 3.4 | 35.7 |
| **330** | 0 | 0 | 0 | 0 | 0 | 5.5 | 0 | 0 | 0 | 0 | 0 | 0 | 0 | 6.9 | 24.1 | 0 |
| **360** | 4.8 | 0 | 23.2 | 0 | 0 | 29.1 | 0 | 8.8 | 0 | 0 | 0 | 0 | 0 | 2.8 | 8.6 | 26.2 |
| **390** | 0 | 0 | 0 | 0 | 0 | 0 | 0 | 0 | 0 | 0 | 0 | 0 | 0 | 0 | 24.1 | 0 |
| **420** | 0 | 0 | 10.7 | 0 | 0 | 21.8 | 0 | 0 | 0 | 0 | 0 | 0 | 0 | 8.3 | 0 | 31.0 |
| **480** | 0 | 0 | 0 | 0 | 0 | 0 | 0 | 0 | 0 | 0 | 0 | 0 | 0 | 0 | 17.2 | 0 |

**Table 8**

| **CONTROLS** | | | | | | | | | | | | | | | |
|---|---|---|---|---|---|---|---|---|---|---|---|---|---|---|---|
| **[s]** | **1** | **2** | **3** | **4** | **5** | **6** | **7** | **8** | **9** | **10** | **11** | **12** | **13** | **14** | **15** |
| **[%]** | | | | | | | | | | | | | | | |
| **5** | 8.6 | 2.7 | 0 | 0 | 0 | 0 | 8.2 | 5.5 | 9.2 | 1.9 | 0 | 8.8 | 0 | 0 | 10.6 |
| **10** | 25.8 | 25.7 | 3.9 | 12.5 | 9.1 | 0 | 22.4 | 20.9 | 34.7 | 1.9 | 9.0 | 13.2 | 0 | 7.1 | 19.7 |
| **15** | 28.0 | 16.2 | 10.4 | 0 | 0 | 0 | 19.4 | 9.1 | 5.1 | 0 | 15.4 | 0, | 0 | 18.6 | 6.1 |
| **20** | 19.4 | 36.5 | 23.9 | 22.9 | 14.5 | 10.5 | 28.6 | 31.8 | 34.7 | 33.3 | 0 | 30.9 | 0 | 44.3 | 30.3 |
| **30** | 12.9 | 14.9 | 35.8 | 14.6 | 38.2 | 33.3 | 18.4 | 19.1 | 14.3 | 27.8 | 33.3 | 35.3 | 27.5 | 25.7 | 24.2 |
| **40** | 5.4 | 4.1 | 19.4 | 10.4 | 23.6 | 50.9 | 1.0 | 8.2 | 2.0 | 7.4 | 19.2 | 8.8 | 47.1 | 4.3 | 9.1 |
| **60** | 0, | 0 | 7.5 | 29.2 | 14.5 | 5.3 | 2.0 | 5.5 | 0 | 20.4 | 17.9 | 2.9 | 25.5 | 0 | 0 |
| **90** | 0, | 0 | 0 | 10.4 | 0 | 0 | 0 | 0 | 0 | 7.4 | 5.1 | 0 | 0 | 0 | 0 |

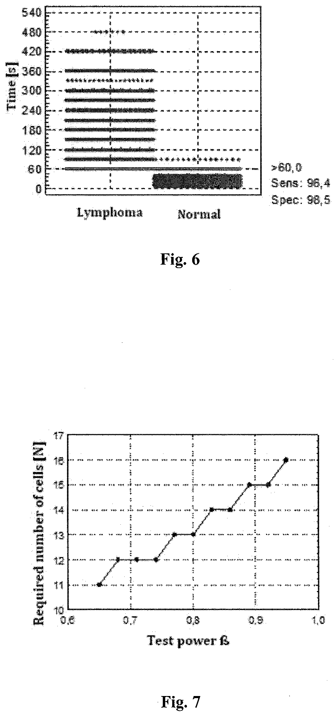

In order to estimate the cut-off value (*cut-off*) for the test differentiating between abnormal and normal cells, the analysis of ROC curves was preformed (Fig. 6).

For a cut-off value of *t* > 60 s, test sensitivity is *Sens.* = 96.4% and specificity *Spec.* = 98.5% (Fig. 7).

The time of stable bond formation by abnormal and normal cells differs significantly regardless of patient's sex and age. For mean (*M*) and standard deviation (*SD*) values for stable cell bonding observed in the study, the minimum number of cells should be *N* = 15. For this number of cells at the significance level pfp = 0.001, the power of the test is β = 0.92 (Fig. 7).

## Claims

1. A method for in vitro diagnosing neoplasms of lymphoid tissues comprising the steps of:
a) providing B lymphocytes previously collected and isolated from a patient,
b) preparing a suspension of B lymphocytes as well as bone marrow stromal cells for measuring by optical tweezers technique,
c) by optical tweezers technique bringing a B lymphocyte to contact with a bone marrow stroma cell, such that the B lymphocyte and the bone marrow stromal cell remain in a direct contact until a stable adhesion is formed,
d) measuring time of the formation of the stable connection (t) between the B lymphocyte and the bone marrow stroma cell, wherein the stable adhesion formation of t > 60 s indicates the presence of neoplastic cells.

2. The method according to claim 1, **characterised in that** before bringing the B lymphocyte into contact with the bone marrow stromal cell, the B lymphocyte is captured into an optical trap by optical tweezers.

3. The method according to claim 2, **characterised in that** the B lymphocyte is captured into the optical trap with the force of 100 pN.

4. The method according to claim 1, **characterised in that** the bond is considered as stable when the B lymphocyte cannot be pulled away from the bone marrow stromal cell using the optical trap generated with a laser power of 200 mW.

5. The method according to claim 1, **characterised in that** the neoplasm of lymphoid tissue is B-cell non-Hodgkin lymphoma (NHL).

6. The method according to claim 1, **characterised in that** the neoplasm of lymphoid tissue is diffuse large B-cell lymphoma (DLBCL), follicular lymphoma (FL), mantle cell lymphoma (MCL), mucosa associated lymphoid tissue lymphoma (MALT), Burkitt lymphoma (BL), and high grade B-cell lymphoma (HGBL).

7. The method according to claim 1, **characterised in that** the B lymphocyte is brought into contact with the central part of the bone marrow stroma cell.

8. The method according to claim 1, **characterised in that** the bone marrow stromal cell is a fibroblast.

9. The method according to claim 1, **characterised in that** bone marrow stromal cells are of HS-5 line.

## Patentansprüche

1. Ein Verfahren zur in vitro Diagnose von Neoplasmen von lymphoiden Geweben, umfassend die Schritte:
a) Bereitstellen von B Lymphozyten, die kürzlich vom Patienten entnommen und isoliert worden sind,
b) Herstellen einer Suspension von B Lymphozyten und Stromazellen des Knochenmarks zum Messen durch optische Pinzetten-Technik,
c) Inkontaktbringen eines B Lymphozyten mit einer Stromazelle des Knochenmarks durch optische Pinzetten-Technik, so dass der B Lymphozyt und die Stromazelle des Knochenmarks in direktem Kontakt bleiben bis sich eine stabile Adhäsion gebildet hat,
d) Messen der Zeit der Bildung einer stabilen Verbindung (t) zwischen dem B Lymphozyten und der Stromazelle des Knochenmarks, wobei die Bildung der stabilen Adhäsion in t> 60 s das Vorliegen von neoplastischen Zellen anzeigt.

2. Das Verfahren nach Anspruch 1, charakterisiert dadurch, dass der B Lymphozyt bevor er mit der Stromazelle des Knochenmarks in Kontakt gebracht wird, durch optische Pinzetten in einer optischen Falle eingefangen wird.

3. Das Verfahren nach Anspruch 2, charakterisiert dadurch, dass der B Lymphozyt in der optischen Falle mit einer Kraft von 100 pN eingefangen wird.

4. Das Verfahren nach Anspruch 1, charakterisiert dadurch, dass das Band als stabil betrachtet wird, wenn der B Lymphozyt nicht von der Stromazelle des Knochenmarks unter Verwendung der optischen Falle, die mit einer Laserkraft von 200 mW erzeugt wird, weggezogen werden kann.

5. Das Verfahren nach Anspruch 1, charakterisiert dadurch, dass das Neoplasma des lymphoiden Gewebes ein B-Zell non-Hodgkin Lymphom (NHL) ist.

6. Das Verfahren nach Anspruch 1, charakterisiert dadurch, dass das Neoplasma des lymphoiden Gewebes ein diffuses großes B-Zell Lymphom (DLBCL), ein follikuläres Lymphom (FL), ein Mantelzelllymphom (MCL), ein Lymphom des Mucosa assoziierten lymphoiden Gewebes (MALT), ein Burkitt Lymphom (BL) und ein hochgradiges B-Zell Lymphom (HGBL) ist.

7. Das Verfahren nach Anspruch 1, charakterisiert dadurch, dass der B Lymphozyt mit dem zentralen Abschnitt der Stromazelle des Knochenmarks in Kontakt gebracht wird.

8. Das Verfahren nach Anspruch 1, charakterisiert dadurch, dass die Stromazelle des Knochenmarks ein Fibroblast ist.

9. Das Verfahren nach Anspruch 1, charakterisiert dadurch, dass die Stromazellen des Knochenmarks aus der HS-5 Linie sind.

## Revendications

1. Procédé de diagnostic in vitro des néoplasmes des tissus lymphoïdes, comprenant les étapes consistant à :
a) fournir des lymphocytes B préalablement prélevés et isolés chez un patient,
b) préparer une suspension de lymphocytes B et de cellules stromales de la moelle osseuse en vue d'une mesure par la technique de la pince optique,
c) par la technique de la pince optique, mettre en contact un lymphocyte B avec une cellule stromale de la moelle osseuse, de telle sorte que le lymphocyte B et la cellule stromale de la moelle osseuse restent en contact direct jusqu'à ce qu'une adhésion stable se forme,
d) mesurer le temps de formation de la liaison stable (t) entre le lymphocyte B et la cellule stromale de la moelle osseuse, la formation d'une adhésion stable de t > 60 s indiquant la présence de cellules néoplasiques.

2. Procédé selon la revendication 1,
**caractérisé en ce qu'**avant de mettre le lymphocyte B en contact avec la cellule stromale de la moelle osseuse, le lymphocyte B est capturé dans un piège optique par la pince optique.

3. Procédé selon la revendication 2,
**caractérisé en ce que** le lymphocyte B est capturé dans le piège optique avec une force de 100 pN.

4. Procédé selon la revendication 1,
**caractérisé en ce que** le lien est considéré comme stable lorsque le lymphocyte B ne peut pas être retiré de la cellule stromale de la moelle osseuse à l'aide du piège optique généré avec une puissance laser de 200 mW.

5. Procédé selon la revendication 1,
**caractérisé en ce que** le néoplasme du tissu lymphoïde est un lymphome non hodgkinien (LNH) à cellules B.

6. Procédé selon la revendication 1,
**caractérisé en ce que** le néoplasme du tissu lymphoïde est un lymphome diffus à grandes cellules B (DLBCL), un lymphome folliculaire (FL), un lymphome à cellules du manteau (MCL), un lymphome du tissu lymphoïde associé aux muqueuses (MALT), un lymphome de Burkitt (BL) et un lymphome à cellules B de haut grade (HGBL).

7. Procédé selon la revendication 1,
**caractérisée en ce que** le lymphocyte B est mis en contact avec la partie centrale de la cellule stromale de la moelle osseuse.

8. Procédé selon la revendication 1,
**caractérisé en ce que** la cellule stromale de la moelle osseuse est un fibroblaste.

9. Procédé selon la revendication 1,
**caractérisé en ce que** les cellules stromales de la moelle osseuse sont de la lignée HS-5.
